# EUROPÄISCHE PATENTSCHRIFT

(11) **EP 2 678 046 B1**
(45) Veröffentlichungstag und Bekanntmachung des Hinweises auf die Patenterteilung: **21.01.2015**
(21) Anmeldenummer: 12706472.3
(22) Anmeldetag: 24.02.2012
(51) Int. Cl.: A61L 12/14, B65B 55/02, B67C 3/26, B67C 7/00, B65D 41/00, B65D 39/00

(54) **DESINFEKTIONSEINRICHTUNG, BEHÄLTER, VERWENDUNG EINES BEHÄLTERS UND DESINFEKTIONSVERFAHREN ZUR DESINFEKTION EINES BEHÄLTERS, INSBESONDERE FÜR EINEN LEBENSMITTELBEHÄLTER**
DISINFECTION APPLIANCE, CONTAINER, USE OF A CONTAINER AND DISINFECTION METHOD FOR DISINFECTING A CONTAINER, IN PARTICULAR FOR A FOOD CONTAINER
DISPOSITIF DE DÉSINFECTION, RÉCIPIENT, UTILISATION D'UN RÉCIPIENT ET PROCÉDÉ DE DÉSINFECTION POUR DÉSINFECTER UN RÉCIPIENT, NOTAMMENT UN RÉCIPIENT ALIMENTAIRE

(30) Priorität: 25.02.2011 DE 102011012394
(43) Veröffentlichungstag der Anmeldung: 01.01.2014
(73) Patentinhaber: Max-Planck-Gesellschaft zur Förderung der Wissenschaften e.V., 80539 München (DE)
(72) Erfinder: MORFILL, Gregor, 81927 München (DE); ZIMMERMANN, Julia, 81925 Munchen (DE)
(74) Vertreter: Gleiss, Alf-Olav
(86) Internationale Anmeldenummer: PCT/EP2012/000808
(87) Internationale Veröffentlichungsnummer: WO 2012/113568

(56) Entgegenhaltungen:
- EP-A1- 2 008 670
- WO-A1-03/077959
- WO-A1-2011/138463
- WO-A2-02/076511
- DE-A1- 19 719 911
- GB-A- 1 592 383
- JP-A- 2006 176 211
- US-A1- 2005 127 843
- US-A1- 2012 037 588
- DATABASE WPI Week 200652 Thomson Scientific, London, GB; AN 2006-505776 XP002674753, & JP 2006 176211 A (DAINIPPON PRINTING CO LTD) 6. Juli 2006 (2006-07-06)
- DATABASE WPI Week 200821 Thomson Scientific, London, GB; AN 2008-C89911 XP002674754, & JP 2008 044640 A (DAINIPPON PRINTING CO LTD) 28. Februar 2008 (2008-02-28)
- DATABASE WPI Week 200966 Thomson Scientific, London, GB; AN 2009-N94139 XP002679538, & JP 2009 218083 A (JIMA KK) 24. September 2009 (2009-09-24)
- DATABASE WPI Week 200534 Thomson Scientific, London, GB; AN 2005-333111 XP002679539, & WO 2005/035825 A1 (KIRIN BREWERY KK) 21. April 2005 (2005-04-21)

## Beschreibung

Die Erfindung betrifft eine Desinfektionseinrichtung zur Desinfektion eines Innenraums eines Behälters, der zur Aufnahme eines Gegenstands, insbesondere einer verderblichen Waren dient, bevorzugt eines Lebensmittelsbehälters. Die Erfindung betrifft auch einen Behälter, insbesondere zur Aufbewahrung einer verderblichen Ware, bevorzugt von Lebensmitteln, mit einer Behälteröffnung, sowie eine Verwendung dieses Behälters.

Bei der Verpackung von Gegenständen, die eine reduzierte Keimlast aufweisen müssen, weil sie beispielsweise verderblich sind, insbesondere von Lebensmitteln, in Behältern muss sichergestellt werden, dass der Innenraum der Behälter vor dem Einfüllen des jeweiligen Gegenstands weitgehend keimfrei ist, damit dieser während der anschließenden Lagerung im geschlossenen Zustand des Behälters aufgrund der verbliebenen Keime nicht vorzeitig verdirbt. Es ist deshalb aus dem Stand der Technik bekannt, insbesondere Lebensmittelbehälter vor dem Befüllen zu sterilisieren, um die Keimlast in den Lebensmittelbehältern auf ein akzeptables Maß zu verringern. Hierzu können die Lebensmittelbehälter mit Wasserstoffperoxid, Persäure und ultravioletter Strahlung behandelt werden, wie beispielsweise in EP 0 411 970 A1 beschrieben wird.

Nachteilig an dieser herkömmlichen Art der Sterilisierung von Behältern ist zunächst die Tatsache, dass zusätzliche Substanzen (z.B. Wasserstoffperoxid, Persäure) benötigt werden, die in aufwendiger Weise bereitgestellt und wieder entsorgt werden müssen.

Ein weiterer Nachteil der vorstehend beschriebenen herkömmlichen Art der Sterilisierung von Behältern ist die unbefriedigende Sterilisierungswirkung.

Schließlich entstehen bei dieser bekannten Art der Sterilisierung Rückstände, was Spülvorgänge erforderlich macht, um die Rückstände zu entfernen.

Der Erfindung liegt deshalb die Aufgabe zugrunde, eine verbesserte Desinfektionseinrichtung bzw. ein entsprechend verbessertes Desinfektionsverfahren zur Desinfektion von Behältern, insbesondere Lebensmittelbehältern zu schaffen. Der Erfindung liegt weiterhin die Aufgabe zugrunde, einen Behälter zu schaffen, der eine verbesserte Desinfektion von in ihm angeordneten Gegenständen ermöglicht. Der Erfindung liegt auch die Aufgabe zugrunde, eine Verwendung eines solchen Behälters anzugeben.

Diese Aufgabe wird durch eine erfindungsgemäße Desinfektionseinrichtung, einen erfindungsgemäßen Behälter, eine erfindungsgemäße Verwendung des Behälters gemäß den unabhängigen Ansprüchen gelöst.

Die Erfindung umfasst die allgemeine technische Lehre, den Innenraum eines Behälters (z.B. eines Lebensmittelbehälters) durch ein nicht-thermisches, atmosphärisches Plasma zu desinfizieren.

Der im Rahmen der Erfindung verwendete Begriff des Desinfizierens ist nicht auf seine fachsprachliche Bedeutung beschränkt, wonach im Rahmen der Desinfektion eine Keimreduktion um einen Faktor von mindestens 10⁵ erfolgt. Vielmehr ist es im Rahmen der Erfindung auch möglich, dass im Rahmen der Desinfektion eine Sterilisation erfolgt, d.h. eine Keimreduktion um einen Faktor von mindestens 10⁶. Die Erfindung beansprucht jedoch auch Schutz für geringere oder größere Keimreduktionen, wobei die akzeptable, nach der Desinfektion verbleibende Keimlast von dem jeweiligen Anwendungsgebiet abhängt.

Bei einer Desinfektion eines Behälters wird der Behälter nach der Desinfektion in der Regel mit dem jeweiligen Gegenstand, insbesondere einer verderblichen Ware, bevorzugt mit einem Lebensmittel befüllt und dann mittels eines Verschlusses (z.B. Deckel, korkenähnlicher Verschluss, Schraubverschluss) verschlossen, um während der anschließenden Lagerung das Eindringen von Keimen zu verhindern. Der Plasmaapplikator der erfindungsgemäßen Desinfektionseinrichtung ist deshalb in einem bevorzugten Ausführungsbeispiel der Erfindung entsprechend dem normalen Verschluss der Behälteröffnung ausgebildet, so dass der Plasmaapplikator anstelle des Verschlusses auf die Behälteröffnung des Behälters aufgesetzt werden kann, um den Innenraum des Behälters zu desinfizieren. Der Plasmaapplikator weist bevorzugt an seiner Innenseite eine Austrittsöffnung auf, um das nicht-thermische, atmosphärische Plasma in den Innenraum des Behälters abzugeben, während der Plasmaapplikator die Behälteröffnung verschließt. Ein solches Ausführungsbeispiel bietet den Vorteil, das sich in dem Innenraum des zu desinfizierenden Behälters eine große Plasmakonzentration mit einer entsprechend guten Desinfektionswirkung realisieren lässt, da die Behälteröffnung durch den Plasmaapplikator verschlossen wird, so dass das Plasma nicht aus dem eigentlichen Wirkungsbereich im Innenraum des Behälters entweichen kann.

Der Verschluss des Plasmaapplikators bildet einen Verschlussdeckel, der auf die Behälteröffnung des zu desinfizierenden Behälters aufgesetzt werden kann. Der Plasmaapplikator kann also beispielsweise einem Schraubdeckel oder einem sonstigen, vorzugsweise herkömmlichen Deckel eines vorzugsweise normalen, handelsüblichen Behälters, insbesondere eines Lebensmittelbehälters, nachempfunden sein.

Hierbei kann es vorteilhaft sein, wenn der als Verschlussdeckel ausgebildete Plasmaapplikator einen Außenquerschnitt aufweist, der sich in Bezug auf den Behälter von außen nach innen verjüngt, so dass der Verschlussdeckel auf verschieden große Behälteröffnungen passt. Der als Verschlussdeckel ausgebildete Plasmaapplikator ragt dann entsprechend dem Querschnitt der Behälteröffnung unterschiedlich tief in den Behälter hinein. Vorzugsweise kann der von dem Plasmaapplikator nachgebildete Verschlussdeckel hierzu einen trapezförmigen Querschnitt aufweisen.

Bei einem anderen Ausführungsbeispiel bildet der von dem Plasmaapplikator nachgebildete Verschluss einen Verschlussstopfen (z.B. korkenähnlich), der in die Behälteröffnung des zu desinfizierenden Behälters eingeführt werden kann. Bei dem Behälter kann es sich also auch um eine Flasche handeln.

Der von dem Plasmaapplikator nachgebildete Verschluss kann also wahlweise auf die Behälteröffnung aufgesetzt und/oder in die Behälteröffnung eingeführt werden. Darüber hinaus bestehen im Rahmen der Erfindung verschiedene weitere Möglichkeiten zum Aufbringen des von dem Plasmaapplikator nachgebildeten Verschlusses auf die Behälteröffnung des zu desinfizierenden Behälters. Beispielsweise kann der Verschluss auf die Behälteröffnung aufgeschraubt bzw. eingeschraubt werden. Die Erfindung ist also hinsichtlich der mechanischen Verbindung zwischen dem durch den Plasmaapplikator nachgebildeten Verschluss und der Behälteröffnung des zu desinfizierenden Behälters nicht auf die vorstehend beschriebenen Beispiele beschränkt.

In einer vorteilhaften Weiterbildung der erfindungsgemäßen Desinfektionseinrichtung weist der Plasmaapplikator eine ringförmige Elektrodenanordnung zur Erzeugung des nicht-thermischen, atmosphärischen Plasmas auf, wobei sich die ringförmige Elektrodenanordnung entlang dem Umfangsrand der Behälteröffnung erstreckt, so dass die ringförmige Elektrodenanordnung den Umfangsrand der Behälteröffnung desinfiziert. Dies ist vorteilhaft, weil der hervorstehende Umfangsrand der Behälteröffnung insbesondere bei Getränkebehältem oftmals einer erhöhten Keimlast ausgesetzt ist und deshalb besonders gründlich desinfiziert werden sollte.

Bei einem bevorzugten Ausführungsbeispiel der Erfindung weist der Plasmaapplikator eine elektrisch betriebene Plasmaquelle zur Erzeugung des nicht-thermischen atmosphärischen Plasmas auf. Derartige elektrisch betriebene Plasmaquellen sind an sich aus dem Stand der Technik bekannt und müssen deshalb nicht näher beschrieben werden. Lediglich beispielhaft wird jedoch auf WO 2010/094304 A1, WO 2010/034451 A1, WO 2010/022871 A1, WO 2008/138504 A1, WO 2007/031250 A1, WO 2010/094307 A1 und EP 10 013 940.1 verwiesen, so dass deren Inhalt der vorliegenden Beschreibung hinsichtlich des Aufbaus und der Funktionsweise einer elektrisch betriebenen Plasmaquelle in vollem Umfang zuzurechnen ist. Die Plasmaquelle kann also beispielsweise eine SMD-Elektrode (SMD: Surface micro discharge), eine SSS-Elektrode (SSS: Self sterilizing surface) oder eine DBD-Elektrode (DBD: Dielectric barrier discharge) zur Plasmaerzeugung aufweisen, wobei derartige Elektroden zur Plasmaerzeugung an sich aus dem Stand der Technik bekannt sind und deshalb nicht näher beschrieben werden müssen.

Darüber hinaus weist der Plasmaapplikator in der erfindungsgemäßen Desinfektionseinrichtung vorzugsweise auch eine integrierte elektrische Energiequelle auf, um die zum Betrieb der Plasmaquelle erforderliche elektrische Energie bereitzustellen.

Bei einem bevorzugten Ausführungsbeispiel der Erfindung handelt es sich bei der elektrischen Energiequelle um einen Piezogenerator, der mechanisch (z.B. manuell) betätigt werden kann und dadurch die zum Betrieb der Plasmaquelle erforderliche elektrische Energie erzeugt. Ein derartiger Piezogenerator erzeugt die elektrische Energie jedoch in der Regel in einem Format (Spannung, Impulsdauer, etc.), das nicht notwendig unmittelbar zur Speisung der Plasmaquelle geeignet ist. Der erfindungsgemäße Plasmaapplikator weist deshalb vorzugsweise auch einen Konverter auf, der eingangsseitig mit dem Piezogenerator und ausgangsseitig mit der Plasmaquelle verbunden ist, wobei der Konverter die von dem Piezogenerator gelieferte elektrische Energie in die für den Betrieb der Plasmaquelle benötigte elektrische Energie umwandelt. Darüber hinaus umfasst der Plasmaapplikator in der erfindungsgemäßen Desinfektionseinrichtung vorzugsweise auch ein Betätigungsglied, das mechanisch mit dem Piezogenerator gekoppelt ist, so dass der Piezogenerator betätigt werden kann, um die zum Betrieb der Plasmaquelle erforderliche elektrische Energie zu erzeugen. Dieses mechanische Betätigungsglied wird vorzugsweise manuell betätigt, indem beispielsweise ein Druckknopf niedergedrückt wird, wie es beispielsweise von herkömmlichen Feuerzeugen im Prinzip bekannt ist. Eine andere Variante eines manuellen Betätigungsgliedes ist eine Drehkurbel, welche den Piezogenerator aktiviert. Die Erfindung ist jedoch nicht auf manuelle Betätigungsglieder beschränkt, sondern grundsätzlich auch mit anderen mechanischen Betätigungsgliedern realisierbar, die von einem Benutzer betätigt werden können. So können beispielsweise auch andere Typen von sogenannten Energy-Harvesting-Energiequellen eingesetzt werden, die beispielsweise in EP 10 013 940.1 beschrieben werden, so dass der Inhalt dieser Druckschrift der vorliegenden Beschreibung hinsichtlich der einsetzbaren Typen von Energy-Harvesting-Energiequellen in vollem Umfang zuzurechnen ist.

Bei der elektrischen Energiequelle zum Betreiben der Plasmaquelle kann es sich jedoch auch um einen elektrischen Generator, eine Batterie (z.B. Akkumulator), eine Solarzelle oder einen Netzanschluss handeln, der mit einem elektrischen Stromnetz verbunden werden kann.

Es wird auch eine Desinfektionsvorrichtung bevorzugt, die sich dadurch auszeichnet, dass der Plasmaapplikator in seiner äußeren Form einem normalen Verschlussdeckel oder einem normalen Verschlussstopfen für einen herkömmlichen Behälter, insbesondere für einen Lebensmittelbehälter, vorzugsweise für eine Flasche, nachempfunden ist. Der Plasmaapplikator kann damit auf einen herkömmlichen, handelsüblichen Behälter aufgesetzt und als Deckel für diesen verwendet werden. Dabei ist es möglich, den Plasmaapplikator ausschließlich zu Desinfektions- oder Sterilisationszwecken auf den Behälter aufzusetzen, ihn anschließend zu entfernen und durch einen normalen, dem Behälter herkömmlicherweise zugeordneten Verschlussdeckel oder Verschlusstopfen zu ersetzen. Es ist jedoch auch möglich, den Plasmaapplikator dauerhaft auf dem Behälter zu belassen, so dass der Behälter durch den Plasmaapplikator dicht verschlossen wird. Bevorzugt ist der Plasmaapplikator dafür auf einen bestimmten Behälter abgestimmt. Er kann beispielsweise als Schraubverschluss ausgebildet sein. Vorzugsweise ist es möglich, den Behälter mit dem Plasmaapplikator luftdicht zu verschließen, wozu dieser ein Dichtelement, beispielsweise eine Gummilippe aufweisen kann.

Es ist auch möglich, dass der Plasmaapplikator als Verschlussdeckel oder -stopfen für verschieden große und/oder verschieden geformte, beispielsweise zylindrische oder eckige Behälter ausgebildet ist. Er ist dann nicht auf einen bestimmten Behälter abgestimmt, sondern vielfältig in Zusammenhang mit verschiedenen Behältern einsetzbar.

Weist der als Verschlussdeckel oder -stopfen ausgebildete Plasmaapplikator einen sich von außen nach innen verjüngenden, vorzugsweise im Wesentlichen trapezförmigen Außenquerschnitt auf, passt er sich mit der sich verjüngenden Gestalt ohne Weiteres verschieden großen Behälteröffnungen an, beispielsweise Flaschen mit verschiedenen Halsdurchmessern, oder eckigen Behältern mit verschieden großer Öffnungsfläche, wobei er mehr oder weniger weit in eine Mündung des Behälters hineinragt, je nach dem an welcher Stelle der Außendurchmesser des Plasmaapplikators mit einem Außendurchmesser der Behältermündung übereinstimmt. VorzugsweiDichtfläche ausgebildet, so dass der Behälter mit dem Plasmaapplikator dicht verschlossen werden kann. Insbesondere ist es möglich, dass die Dichtfläche ein elastisches Material, beispielsweise Gummi, umfasst, um eine Dichtwirkung zu erzielen. Besonders bevorzugt ist der Behälter mithilfe des Plasmaapplikators luftdicht verschließbar.

Es wird auch noch eine nicht zur Erfindung gehörende Desinfektionseinrichtung zur Desinfektion von Getränkebehältern, insbesondere Gläsern, Babyflaschen und/oder Bierkrügen beschrieben, wobei die Desinfektionseinrichtung eine Aufnahme aufweist, in der ein Stempel verlagerbar ist. Die Aufnahme ist vorzugsweise wannenförmig ausgebildet. Der Stempel umfasst eine Plasmaquelle zumindest in einem Bereich, welcher mit einem Mündungsrand eines zu desinfizierenden Behälters zusammenwirkt. Demnach ist die Plasmaquelle bevorzugt ringförmig ausgebildet und/oder umfasst mindestens eine ringförmig ausgebildete Elektrode. Die Plasmaquelle ist durch Verlagerung des Stempels aktivierbar. Ein Getränkebehälter wird bevorzugt mit der Mündung nach unten auf den Stempel augesetzt. Dann wird der Stempel vorzugsweise durch einen Druck auf den Getränkebehälter verlagert, und die Plasmaquelle wird aktiviert, so dass insbesondere die Mündung des Behälters durch das entstehende Plasma behandelt, insbesondere desinfiziert wird.

Ähnliche Vorrichtungen sind für Getränkebehälter, insbesondere für Bierkrüge bekannt, wobei bei einem Druck auf einen Stempel Wasser in den Bierkrug gespritzt wird. Bei der vorliegenden, nicht zur Erfindung gehörenden Desinfektionseinrichtung wird stattdessen ein Plasma erzeugt, wodurch insbesondere die Mündung des Getränkebehälters effizient desinfiziert beziehungsweise zugsweise den Stempel, die Plasmaquelle und besonders bevorzugt eine elektrische Energiequelle für die Plasmaquelle. Diese ist durch eine Verlagerung des Stempels aktivierbar, so dass sie dann die zur Erzeugung des Plasmas nötige Energie an die Plasmaquelle abgibt.

Die Erfindung umfasst auch einen Behälter nach Anspruch 10, der insbesondere zur Aufbewahrung von Lebensmitteln, aber auch anderen verderblichen Waren und/oder anderen Gegenständen, deren Keimlast reduziert oder klein gehalten werden soll, dient. Dieser weist eine Behälteröffnung auf. Der Behälter zeichnet sich dadurch aus, dass er einen die Behälteröffnung verschließenden Verschlussdeckel oder einen Verschlussstopfen umfasst, der als Desinfektionseinrichtung nach einem der zuvor beschriebenen Ausführungsbeispiele ausgebildet ist. Der Behälter kann zur Reduzierung der Keimlast von in ihm aufgenommenen Gegenständen vorgesehen sein. Dabei wird bei einer Aktivierung des als Plasmaapplikator ausgebildeten Verschlussdeckels oder Verschlussstopfens nicht nur ein Innenraum des Behälters desinfiziert, sondern auch eine zugängliche Oberfläche des in dem Behälter angeordneten Gegenstands. Um diese zu vergrößern, ist bevorzugt vorgesehen, dass ein Boden des Behälters Ausnehmungen oder Vorsprünge umfasst, durch welche das nicht-thermische, atmosphärische Plasma Oberflächenbereiche an einer Unterseite des in dem Behälter angeordneten Gegenstands erreichen kann.

Um die Keimlast auf einem Gegenstand zu reduzieren, insbesondere diesen zu desinfizieren oder zu sterilisieren, ist es möglich, den Gegenstand in dem Behälter anzuordnen. Er kann dann nach der Plasmabehandlung wiederum entnommen werden. Es ist aber auch möglich, einen Gegenstand, insbesondere verderbliche Ware, bevorzugt Lebensmittel, in dem Behälter zu transportieren und/oder zu lagern.

Zur Erfindung gehört auch eine Verwendung des zuvor beschriebenen Behälters zur Reduzierung der Keimlast auf einem Gegenstand, insbesondere auf verderblicher Ware, bevorzugt auf Lebensmitteln. Hierzu wird - wie zuvor beschrieben - der Gegenstand, insbesondere die verderbliche Ware oder das Lebensmittel in dem Behälter angeordnet und anschließend in diesem desinfiziert. Es ist dann möglich, den Gegenstand zumindest teilweise aus dem Behälter zu entnehmen, oder ihn in dem Behälter zu lagern und/oder zu transportieren.

Die Erfindung umfasst auch ein Desinfektionsverfahren zur Desinfektion eines Innenraums eines Behälters, der zur Aufnahme eines Gegenstands, insbesondere einer verderblichen Ware dient, wobei der Behälter bevorzugt ein Lebensmittelbehälter ist. Das Verfahren zeichnet sich dadurch aus, dass ein nicht-thermisches, atmosphärisches Plasma in den Innenraum des Behälters appliziert wird, so dass das Plasma diesen desinfiziert.

Insbesondere wird ein Desinfektionsverfahren bevorzugt, welches folgende Schritte umfasst: Der Behälter wird mit einem Gegenstand, insbesondere einer verderblichen Ware, bevozugt mit einem Lebensmittel befüllt. Vor dem Befüllen des Behälters mit dem Gegenstand und/oder nach dem Befüllen des Behälters mit dem Gegenstand wird in den Innenraum des Behälters Plasma appliziert, so dass entweder der Innenraum des Behälters allein oder auch der in dem Behälter angeordnete Gegenstand plasmabehandelt, insbesondere desinfiziert wird. Der Plasmapplikator wird dann abgenommen, und die Behälteröffnung wird mit einem normalen Verschluss des befüllten Behälters für eine Lagerung und/oder einen Transport verschlossen. Dieses Verfahren wird insbesondere dann durchgeführt, wenn der Plasmaapplikator als Verschlussdeckel oder -stopfen für verschiedene Behälter ausgebildet ist, so dass er zwischen verschiedenen Behältern ausgetauscht werden kann. Typischerweise ist dann ein Plasmaapplikator für mehrere Behälter vorgesehen, so dass er abgenommen und für weitere Behälter verwendet wird.

Es wird auch ein nicht zur Erfindung gehörendes Verfahren beschrieben, welches folgende Schritte umfasst: Der Behälter wird mit einem Gegenstand, insbesondere einer verderblichen Ware, bevorzugt mit einem Lebensmittel befüllt. Der Behälter wird mit einem als Verschlussdeckel oder Verschlussstopfen für den Behälter ausgebildeten Plasmaapplikator verschlossen. Das Plasma wird vor dem Befüllen des Behälters mit dem Gegenstand und/oder nach dem Befüllen des Behälters mit dem Gegenstand in den Innenraum des Behälters appliziert. Es ist möglich, dass der leere Behälter zunächst verschlossen wird, wonach Plasma appliziert wird, und dass der Behälter dann erneut geöffnet wird, um den Gegenstand einzulegen. Danach wird der Behälter erneut verschlossen, und der befüllte Behälter wird gegebenenfalls erneut mit Plasma beaufschlagt.

Es ist auch möglich, dass der leere Behälter nicht plasmabehandelt wird, sondern dass die Plasmabehandlung erst erfolgt, wenn der Gegenstand in dem Behälter angeordnet ist.

Wesentlich ist bei diesem Verfahren, dass der als Verschlussdeckel oder Verschlusstopfen für den Behälter ausgebildete Plasmaapplikator nach dem Applizieren des Plasmas und/oder nach dem Befüllen des Behälters auf demselben verbleibt, so dass der Gegenstand, insbesondere die verderbliche Ware in dem mit dem als Verschlussdeckel oder Verschlussstopfen ausgebildeten Plasmaapplikator verschlossenen Behälter gelagert und/oder transportiert wird. In diesem Fall ist dem Behälter also vorzugsweise ein passender Verschlussdeckel oder Verschlussstopfen in Form eines Plasmaapplikators zugeordnet, und der Plasmaapplikator verbleibt als Deckel oder Stopfen beim Behälter und verschließt diesen dicht, vorzugsweise luftdicht, auch während einer Lagerung und/oder während eines Transports.

Andere vorteilhafte Weiterbildungen der Erfindung sind in den Unteransprüchen gekennzeichnet oder werden nachstehend zusammen mit der Beschreibung der bevorzugten Ausführungsbeispiele der Erfindung anhand der Figuren näher erläutert. Es zeigen:
- Figur 1: eine schematische Querschnittsansicht einer erfindungsgemäßen Desinfektionseinrichtung mit einem deckelförmigen Plasmaapplikator, der auf eine Öffnung eines Behälters aufgesetzt werden kann,
- Figur 2: eine Abwandlung der Desinfektionseinrichtung aus Figur 1 mit einer Batterie anstelle eines Piezogenerators zum Betrieb der Plasmaquelle,
- Figur 3: eine Abwandlung der Desinfektionseinrichtung aus Figur 3 mit einem elektrischen Generator zum Betrieb der Plasmaquelle,
- Figur 4: eine Abwandlung der Desinfektionseinrichtung aus Figur 1 mit einem Netzanschluss anstelle des Piezogenerators,
- Figur 5: eine Abwandlung der Desinfektionseinrichtung aus Figur 1 mit einem im Querschnitt trapezförmigen Deckel,
- Figur 6: eine Abwandlung der Desinfektionseinrichtung aus Figur 1 mit einer in den Deckel integrierten ringförmigen Elektrodenanordnung zur Desinfektion des Umfangsrandes der Behälteröffnung,
- Figur 7A: eine vereinfachte Querschnittsansicht einer erfindungsgemäßen Desinfektionseinrichtung in Form eines Verschlussstopfens,
- Figur 7B: eine Querschnittsansicht einer Öffnung eines Behälters für den Verschlussstopfen gemäß Figur 7A,
- Figur 7C: die Desinfektionseinrichtung gemäß den Figuren 7A und 7B, wobei der Verschlusstopfen in die Behälteröffnung eingesetzt ist,
- Figur 8: das erfindungsgemäße Desinfektionsverfahren in Form eines Flussdiagramms,
- Figur 9: eine schematische Darstellung eines alternativen Ausführungsbeispiels einer erfindungsgemäßen Desinfektionseinrichtung für einen beutelförmigen Lebensmittelbehälter,
- Figuren 10A-10C: verschiedene Verfahrensstadien bei der Desinfektion, Befüllung und Lagerung eines schlauchförmigen Lebensmittelbehälters,
- Figur 11: eine Abwandlung des Ausführungsbeispiels aus Figur 1.
- Figuren 12A und 12B: eine Desinfektionseinrichtung für Getränkebehälter.

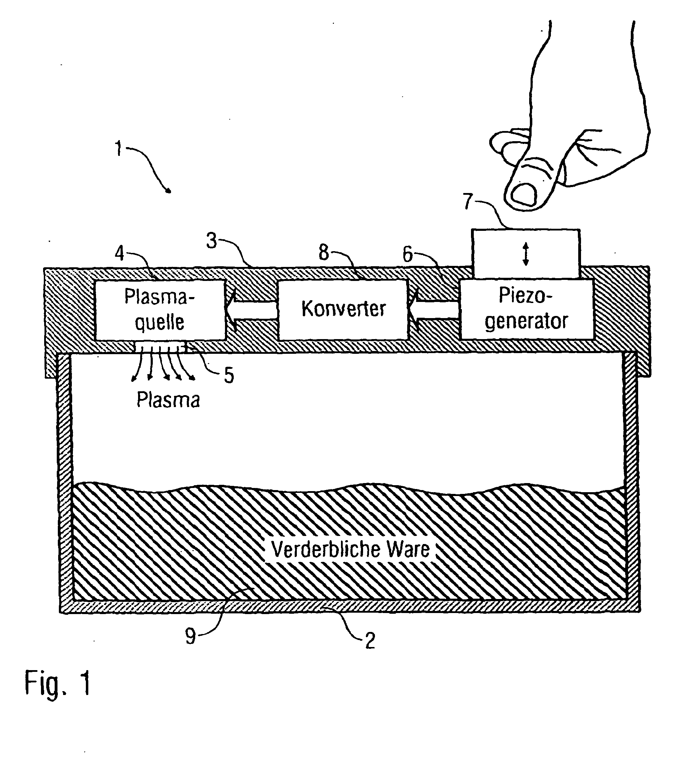

Figur 1 zeigt eine vereinfachte Querschnittsdarstellung einer erfindungsgemäßen Desinfektionseinrichtung 1, die zur Desinfektion eines Behälters 2, insbesondere eines Lebensmittelbehälters dient, wobei der Behälter 2 beispielsweise aus Kunststoff bestehen kann und typischerweise wannenförmig ausgebildet ist, so dass er an seiner Oberseite eine Behälteröffnung aufweist. Die Erfindung ist keinesfalls eingeschränkt auf den Lebensmittelbereich. Jeder andere Gegenstand, dessen Keimlast reduziert oder klein gehalten werden soll, ist in einem entsprechend geeigneten Behälter behandelbar.

Die Desinfektionseinrichtung 1 besteht im Wesentlichen aus einem Plasmaapplikator 3, der in seiner äußeren Form dem normalen Verschlussdeckel für den Behälter 2 nachempfunden ist, so dass der Plasmaapplikator 3 anstelle des normalen Verschlussdeckels auf die Behälteröffnung des Behälters 2 aufgesetzt werden kann. Vorzugsweise ist der Plasmaapplikator 3 hier dem Behälter 2 fest zugeordnet. Er ersetzt also besonders bevorzugt dessen gewöhnlichen Deckel.

In dem verschlussdeckelförmigen Plasmaapplikator 3 ist eine elektrisch betriebene Plasmaquelle 4 integriert, die ein nicht-thermisches, atmosphärisches Plasma durch eine Austrittsöffnung 5 in den Innenraum des Behälters 2 appliziert, wenn der verschlussdeckelförmige Plasmaapplikator 3 auf die Behälteröffnung des Behälters 2 aufgesetzt ist.

Die elektrisch betriebene Plasmaquelle 4 wird von einem Piezogenerator 6 mit der zum Betrieb erforderlichen elektrischen Energie versorgt, wobei der Piezogenerator 6 durch ein manuelles Betätigungsglied 7 in Form eines Druckknopfs aktiviert werden kann. Hierzu drückt ein Benutzer den Druckknopf in Pfeilrichtung nieder, woraufhin der Piezogenerator 6 die zum Niederdrücken des Betätigungsglieds 7 erforderliche mechanische Energie in elektrische Energie umwandelt.

Die von dem Piezogenerator 6 gelieferte elektrische Energie eignet sich jedoch in der Regel nicht unmittelbar zum Speisen der Plasmaquelle 4. Zwischen dem Piezogenerator 6 und der Plasmaquelle 4 ist deshalb vorzugsweise ein Konverter 8 angeordnet, der die von dem Piezogenerator 6 gelieferte elektrische Energie hinsichtlich ihres Formats so umwandelt, dass sie zum Speisen der Plasmaquelle 4 geeignet ist, wobei der Konverter 8 die elektrische Energie auch zwischenspeichern kann.

In der Zeichnung ist der Plasmaapplikator 3 in aufgesetztem Zustand dargestellt, wobei der Behälter 2 bereits mit einem Gegenstand, vorzugsweise mit verderblicher Ware, insbesondere mit einem Lebensmittel 9 befüllt ist. In diesem Zustand desinfiziert der Plasmaapplikator 3 also die Oberseite des Lebensmittels 9 unmittelbar vor dem Verschließen des Behälters 2 mit dem eigentlichen Verschlussdeckel. Es ist jedoch zu erwähnen, dass der Innenraum des Behälters 2 in der gleichen Weise bevorzugt auch vor dem Befüllen mit dem Lebensmittel 9 desinfiziert wird, wie noch detailliert beschrieben wird.

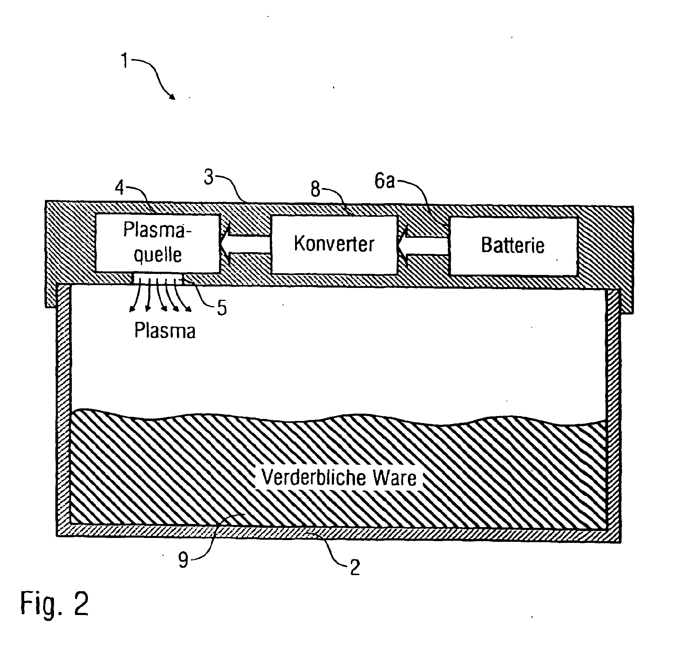

Figur 2 zeigt eine Abwandlung der Desinfektionseinrichtung gemäß Figur 1, so dass zur Vermeidung von Wiederholungen auf die vorstehende Beschreibung verwiesen wird, wobei für entsprechende Einzelheiten dieselben Bezugszeichen verwendet werden.

Eine Besonderheit dieses Ausführungsbeispiels besteht darin, dass anstelle des Piezogenerators 6 eine elektrische Batterie 6a vorgesehen ist, um die elektrisch betriebene Plasmaquelle 4 zu speisen.

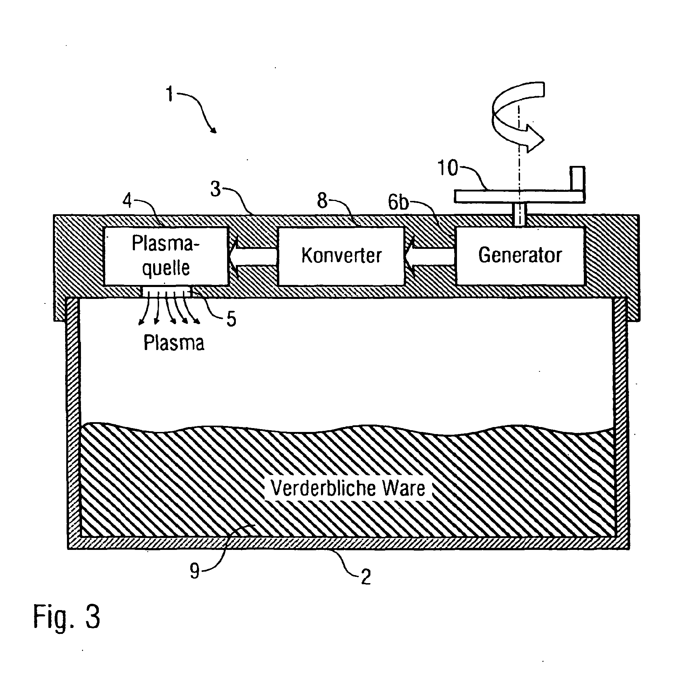

Auch das Ausführungsbeispiel gemäß Figur 3 stimmt weitgehend mit dem vorstehend beschriebenen und in Figur 1 dargestellten Ausführungsbeispiel überein, so dass zur Vermeidung von Wiederholungen auf die vorstehende Beschreibung verwiesen wird, wobei für entsprechende Einzelheiten dieselben Bezugszeichen verwendet werden.

Eine Besonderheit dieses Ausführungsbeispiels besteht darin, dass zur elektrischen Energieversorgung anstelle des Piezogenerators 6 ein mechanischer Generator 6b vorgesehen ist, der durch eine Kurbel 10 angetrieben werden kann, um die zum Betrieb der Plasmaquelle 4 erforderliche elektrische Energie zu erzeugen.

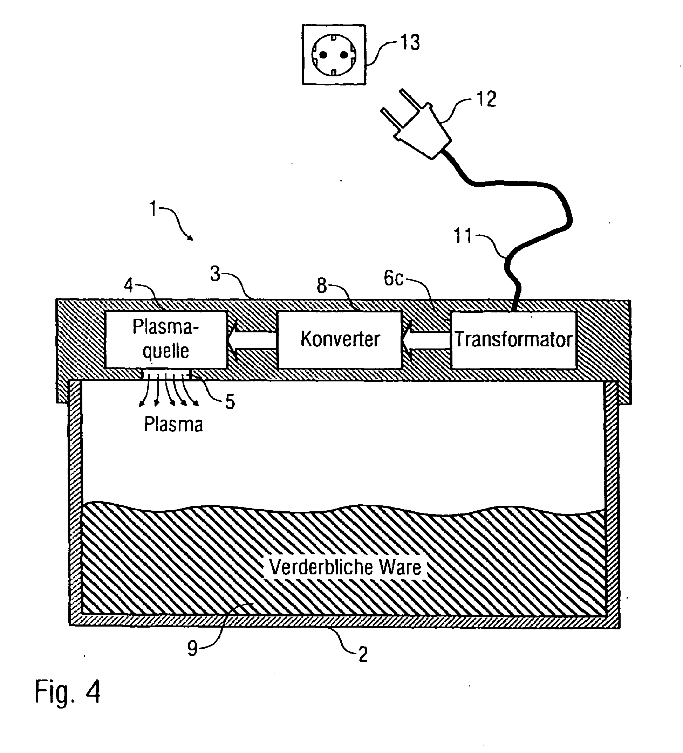

Das Ausführungsbeispiel gemäß Figur 4 stimmt ebenfalls weitgehend mit dem vorstehend beschriebenen und in Figur 1 dargestellten Ausführungsbeispiel überein, so dass zur Vermeidung von Wiederholungen auf die vorstehende Beschreibung verwiesen wird, wobei für entsprechende Einzelheiten dieselben Bezugszeichen verwendet werden.

Eine Besonderheit dieses Ausführungsbeispiels besteht darin, dass anstelle des Piezogenerators 6 zum Speisen der Plasmaquelle 4 ein Transformator 6c vorgesehen ist, der über ein Netzkabel 11 und einen Netzstecker 12 mit einem herkömmlichen Stromnetz verbunden werden kann, indem der Netzstecker 12 in eine entsprechende Steckdose 13 eingesteckt wird.

Besonders bevorzugt sind die in den Figuren 1 bis 4a dargestellten Ausführungsbeispiele als Behälter 2 ausgebildet, der eine Behälteröffnung und einen diese verschließenden Verschlussdeckel oder Verschlussstopfen umfasst, der als Desinfektionseinrichtung, insbesondere als Plasmaapplikator 3 ausgebildet ist. Bei den Ausführungsbeispielen gemäß den Figuren 1 bis 4 ist also bevorzugt der Plasmaapplikator 3 dem Behälter 2 als Verschlussdeckel oder Verschlussstopfen fest zugeordnet. Er ersetzt insoweit den ansonsten vorgesehenen Verschlussdeckel oder Verschlussstopfen ohne Plasma-Funktion. Der Behälter 2 gemäß den Figuren 1 bis 4 wird demnach vorzugsweise durch den Plasmaapplikator 3 dauerhaft luftdicht verschlossen, so dass ein in dem Behälter angeordneter Gegenstand darin gelagert und/oder transportiert werden kann.

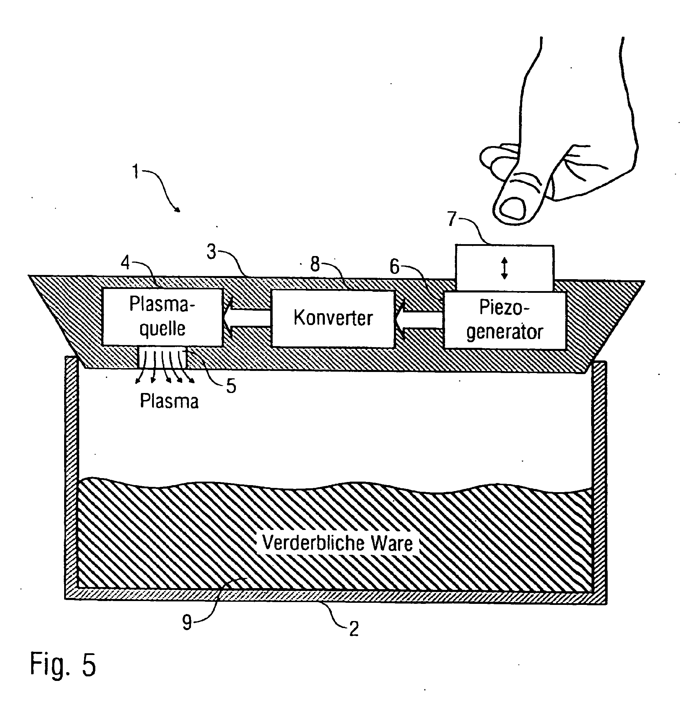

Figur 5 zeigt eine Abwandlung des Ausführungsbeispiels gemäß Figur 1, so dass zur Vermeidung von Wiederholungen auf die vorstehende Beschreibung verwiesen wird, wobei für entsprechende Einzelheiten dieselben Bezugszeichen verwendet werden.

Eine Besonderheit dieses Ausführungsbeispiels besteht darin, dass der einem Verschlussdeckel für den Behälter 2 nachempfundene Plasmaapplikator 3 einen im Wesentlichen trapezförmigen Außenquerschnitt aufweist. Dies bietet den Vorteil, dass der Plasmaapplikator 3 auf unterschiedlich große Behälteröffnungen aufgesetzt werden kann, wobei er sich an die Größe einer Behälteröffnung anpasst, indem er entsprechend tief in den Behälter 2 hineinragt.

Im Unterschied zu den Figuren 1 bis 4 ist der Plasmaapplikator 3 bei dem Ausführungsbeispiel gemäß Figur 5 dem Behälter 2 nicht fest zugeordnet, sonder stattdessen so ausgebildet, dass er für verschiedene Behälter 2 verwendbar ist.

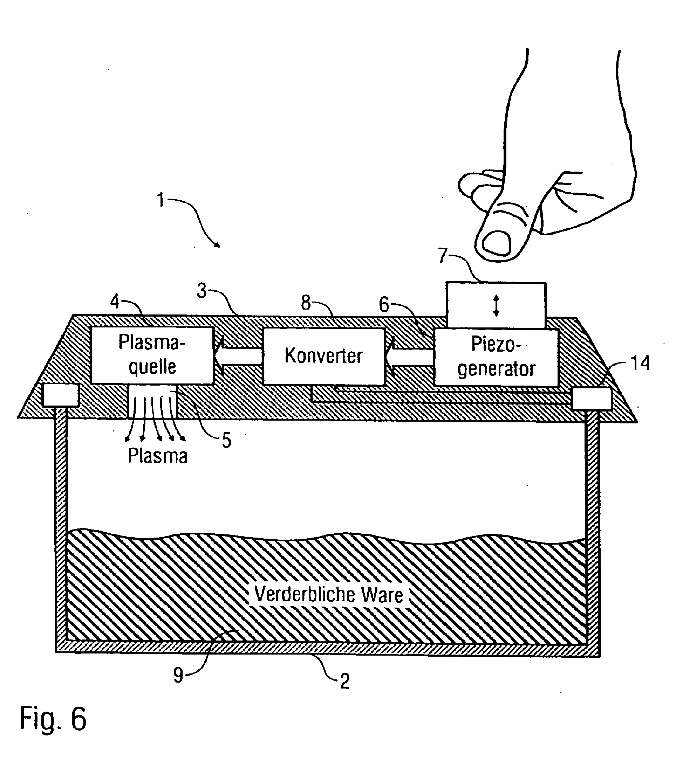

Figur 6 ist eine Abwandlung des Ausführungsbeispiels gemäß Figur 1, so dass zur Vermeidung von Wiederholungen wieder auf die vorstehende Beschreibung verwiesen wird, wobei für entsprechende Einzelheiten dieselben Bezugszeichen verwendet werden.

Eine Besonderheit dieses Ausführungsbeispiels besteht darin, dass der Plasmaapplikator 3 zusätzlich zu der Plasmaquelle 4 eine ringförmige Elektrodenanordnung 14 aufweist, die sich entlang dem Umfangsrand der Behälteröffnung des Behälters 2 erstreckt und ein nicht-thermisches atmosphärisches Plasma erzeugt, um den Umfangsrand zu sterilisieren bzw. zu desinfizieren.

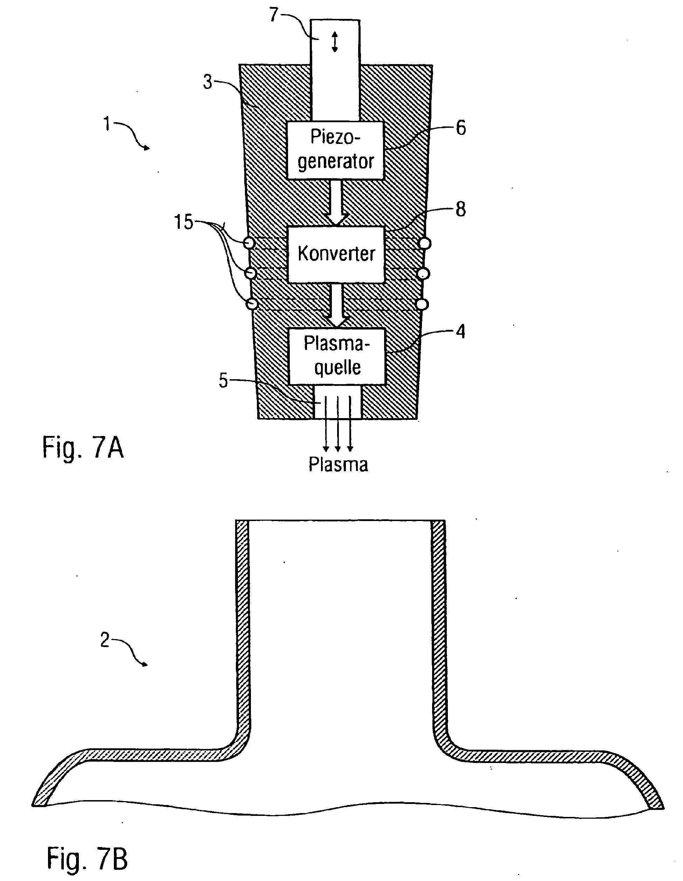

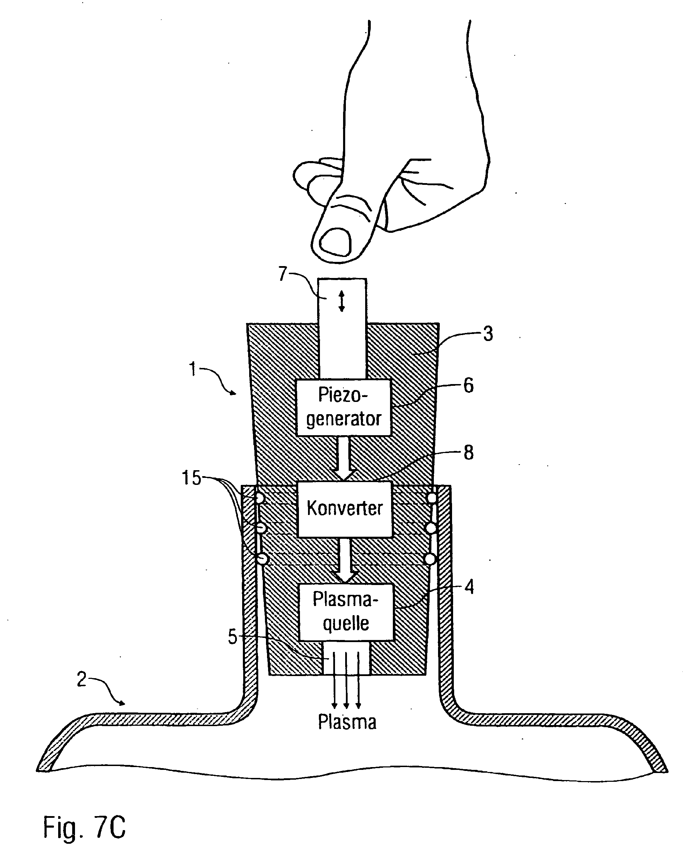

Die Figuren 7A-7C zeigen ein anderes Ausführungsbeispiel einer erfindungsgemäßen Desinfektionseinrichtung 1, wobei dieses Ausführungsbeispiel teilweise mit den vorstehend beschriebenen Ausführungsbeispielen übereinstimmt, so dass zur Vermeidung von Wiederholungen auf die vorstehende Beschreibung verwiesen wird, wobei für entsprechende Einzelheiten dieselben Bezugszeichen verwendet werden.

Ein grundsätzlicher Unterschied dieses Ausführungsbeispiels gegenüber den vorstehend beschriebenen Ausführungsbeispielen besteht darin, dass der Plasmaapplikator 3 nicht einem Verschlussdeckel für den Behälter 2 nachempfunden ist, sondern einem Verschlussstopfen. Der Plasmaapplikator 3 wird also entsprechend einem Verschlussstopfen in die Behälteröffnung des Behälters 2 eingesetzt, um den Behälter 2 zu desinfizieren, wobei es sich bei dem Behälter 2 beispielsweise um eine Flasche handeln kann.

Auch in diesem Fall ist es möglich, dass der Plasmaapplikator 3 dem Behälter 2 fest zugeordnet ist, so dass der Behälter 2 dauerhaft mit dem Plasmaapplikator 3 vorzugsweise luftdicht verschlossen wird. Bei einem anderen Ausführungsbeispiel ist es jedoch möglich, dass der Plasmaapplikator 3 passend für verschiedene Behälter 2 ausgebildet ist, so dass diese letztlich nach einer Verwendung des Plasmaapplikators 3 durch herkömmliche Verschlussstopfen verschlossen werden.

Eine weitere Besonderheit besteht darin, dass der stopfenförmige Plasmaapplikator 3 an seiner konischen Mantelfläche mehrere Dichtringe 15 aufweist, um die Mantelfläche des stopfenförmigen Plasmaapplikators 3 gegenüber der Innenwand der Behälteröffnung des Behälters 2 abzudichten.

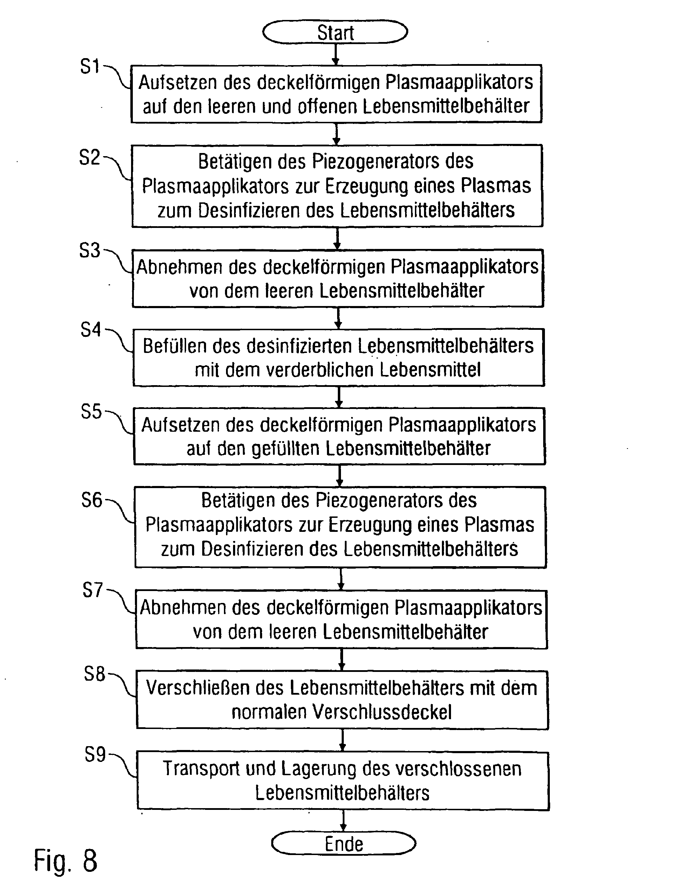

Im Folgenden wird nun das in Figur 8 dargestellte Flussdiagramm erläutert, welches eine Ausführungsform des erfindungsgemäßen Desinfektionsverfahrens beschreibt.

In einem ersten Schritt S1 wird zunächst der deckelförmige Plasmaapplikator 3 auf den leeren und offenen Behälter 2 aufgesetzt, der in Figur 8 beispielhaft als Lebenmittelbehälter bezeichnet ist. Anschließend wird dann in einem Schritt S2 der Piezogenerator 6 des Plasmaapplikators 3 betätigt, um ein nicht-thermisches, atmosphärisches Plasma zum Desinfizieren des Innenraums des Behälters 2 zu erzeugen.

Nach der so erfolgenden Desinfektion des Behälters 2 wird der Plasmaapplikator 3 dann in einem Schritt S3 von dem leeren Behälter 2 abgenommen.

Daraufhin wird der noch leere Behälter 2 dann in einem Schritt S4 mit einem Gegenstand, insbesondere einer verderblichen Ware, bevorzugt mit dem Lebensmittel 9 befüllt.

Anschließend erfolgt dann in einem Schritt S5 wieder ein Aufsetzen des deckelförmigen Plasmaapplikators 3 auf den dann gefüllten Behälter 2.

In einem nächsten Schritt S6 wird dann wieder der Piezogenerator 6 betätigt, um das nicht-thermische, atmosphärische Plasma zu erzeugen, wodurch die Oberfläche des eingefüllten Gegenstands, insbesondere des Lebensmittels 9 desinfiziert wird.

Im nächsten Schritt S7 wird der Plasmaapplikator 3 dann von dem Behälter 2 abgenommen.

Im nächsten Schritt S8 wird der Behälter 2 dann mit einem normalen Verschlussdeckel verschlossen, woraufhin dann in einem letzten Schritt S9 ein Transport und/oder eine Lagerung des Behälters 2 erfolgen kann.

Wesentlich ist, dass das Verfahren nicht auf eine Anwendung in Zusammenhang mit verderblichen Lebensmitteln beschränkt ist. Auch jeder andere Gegenstand, dessen Keimlast reduziert werden soll, oder dessen Keimlast klein gehalten werden soll, kann in dem Behälter 2 angeordnet und dort mit Plasma behandelt werden. Die konkrete Erwähnung eines Lebensmittelbehälters und eines verderblichen Lebensmittels ist daher ausschließlich als besonders bevorzugte Ausführungsform des Verfahrens zu verstehen.

Es ist möglich, die Schritte S1 und S2 wegzulassen, also den Behälter 2 nicht selbstständig ohne eingebrachten Gegenstand zu desinfizieren. Stattdessen ist es möglich, dass das Verfahren mit dem Schritt S3 beginnt und lediglich der befüllte Behälter 2 samt seinem Inhalt mit dem Plasma behandelt wird.

Es ist auch möglich, den Schritt S2 durchzuführen und den Schritt S6 wegzulassen, insbesondere wenn ein bereits desinfizierter oder sterilisierter Gegenstand 2 in den Behälter eingelegt wird.

Jedenfalls hat eine Sterilisation oder Desinfektion des Behälters 2 vor dem Einlegen des Gegenstands den Vorteil, dass dieser nicht durch eine Berührung mit einer möglicherweise kontaminierten Wandung des Behälters 2 selbst kontaminiert wird.

Die hier beschriebene Ausführungsform des Verfahrens findet bevorzugt dann Anwendung, wenn der Plasmaapplikator 3 dem Behälter 2 nicht fest zugeordnet sondern für verschiedene Behälter 2 geeignet ist.

Ist dagegen der Behälter 2 selbst mit einem Verschlussdeckel oder Verschlussstopfen ausgestattet, der als Plasmaapplikator 3 ausgebildet ist, ist also der Plasmaapplikator 3 in diesem Fall dem Behälter 2 fest als Verschlussdeckel oder Verschlussstopfen zugeordnet, wird das Verfahren bevorzugt mit folgender Modifikation durchgeführt: In diesem Fall werden die Schritte S7 und S8 weggelassen, und der Behälter 2 bleibt bevorzugt mit dem als Verschlussdeckel oder Verschlussstopfen ausgebildeten Plasmaapplikator 3 verschlossen. Ein Transport und/oder eine Lagerung des Behälters 2 erfolgt also, während dieser mit dem als Plasmaapplikator 3 ausgebildeten Verschlussdeckel oder Verschlussstopfen verschlossen ist.

Besonders bevorzugt ist es möglich, den Behälter 2 wieder zu öffnen, wenn zumindest ein Teil der darin gelagerten Ware entnommen werden soll. Beispielsweise ist es möglich, Lebensmittelreste in dem Behälter 2 aufzubewahren und zur Lagerung zu desinfizieren beziehungsweise zu sterilisieren. Nach einer gewissen Lagerzeit kann zumindest ein Teil der Lebensmittelreste entnommen werden, und der Behälter 2 kann zur weiteren Lagerung wieder mit dem Plasmaapplikator 3 verschlossen werden. Dabei wird vorzugsweise erneut eine Plasmabehandlung des verbleibenden Rests der gelagerten Ware durchgeführt, so dass diese erneut sterilisiert beziehungsweise desinfiziert und damit letztlich lagerfähig gemacht wird. Insbesondere können so beim Öffnen in den Behälter 2 eingedrungene Keime dezimiert beziehungsweise unschädlich gemacht werden.

Es zeigt sich, dass das hier beschriebene Verfahren im Haushaltsbereich durchführbar ist. Entsprechend ist/sind auch der Behälter und/oder die Desinfektionseinrichtung im Wesentlichen im Haushaltsbereich einsetzbar. Es ist auch möglich, das Verfahren, die Desinfektionseinrichtung und den Behälter im unternehmerischen Bereich, beispielsweise der Gastronomie oder im Lebensmittelfachhandel, beispielsweise in einer Metzgerei einzusetzen.

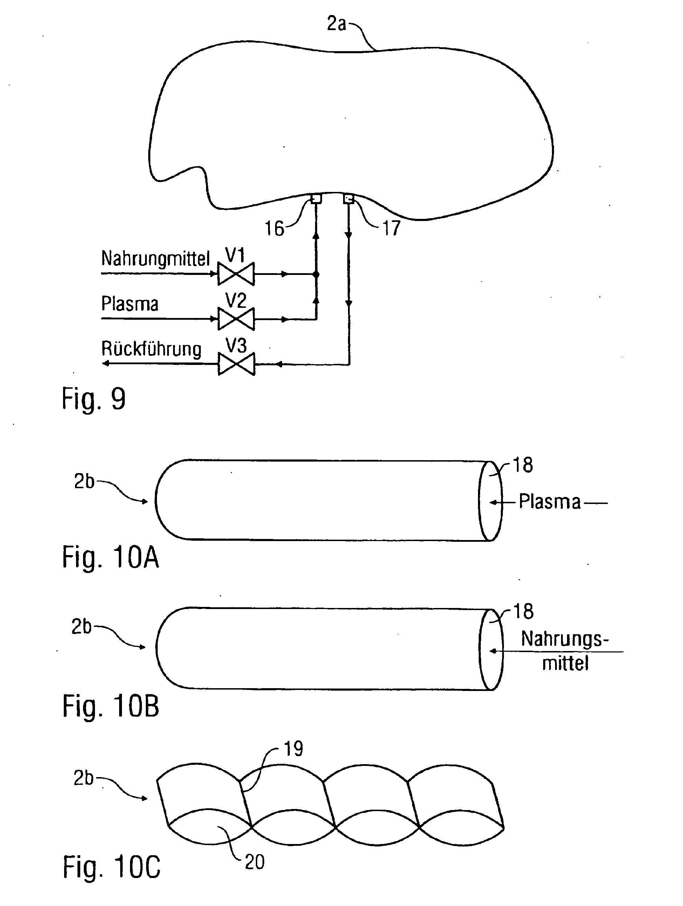

Figur 9 zeigt ein alternatives Ausführungsbeispiel einer erfindungsgemäßen Desinfektionseinrichtung insbesondere für den industriellen Bereich, wobei ein beutelförmiger Behälter 2a vorgesehen ist, der über eine Eintrittsöffnung 16 mit einem Gegenstand, vorzugsweie mit einem Nahrungsmittel befüllt werden kann, wobei eine Entnahme des Nahrungsmittels über eine Austrittsöffnung 17 möglich ist. Der beutelförmige Behälter 2a eignet sich also beispielsweise als Lebensmittelbehälter, insbesondere für flüssige oder pastöse Lebensmittel, beziehungsweise ist bevorzugt als solcher ausgebildet.

Die Eintrittsöffnung 16 und die Austrittsöffnung 17 sind über Ventile V1, V2, V3 mit einer Zuführung für den Gegenstand, vorzugsweise das einzufüllende Nahrungsmittel, und mit einer Zuführung für ein nicht-thermisches, atmosphärisches Plasma verbunden. Vor dem Befüllen des beutelförmigen Behälters 2a mit dem Gegenstand, beispielsweise mit dem verderblichen Nahrungsmittel, über das Ventil V1 kann der beutelförmige Behälter 2a also über das Ventil V2 zunächst mit dem nicht-thermischen, atmosphärischen Plasma befüllt werden, um den Innenraum des beutelförmigen Behälters 2a zu desinfizieren.

Die Figuren 10A-10C zeigen schließlich einen schlauchförmigen Behälter 2b insbesondere für den industriellen Bereich, der in dem in Figur 10A dargestellten ersten Schritt zunächst über eine Einfüllöffnung 18 mit einem nicht-thermischen, atmosphärischen Plasma befüllt wird, um den Innenraum des schlauchförmigen Behälters 2b zu desinfizieren.

Anschließend wird der schlauchförmige Behälter 2b dann in dem in Figur 10B dargestellten Verfahrensschritt über die Einfüllöffnung 18 mit einem Gegenstand, beispielsweise mit dem eigentlichen Nahrungsmittel befüllt. Der schlauchförmige Behälter 2b ist vorzugsweise als Lebensmittelbehälter ausgebildet.

In einem weiteren Verfahrensschritt, der in Figur 10C dargestellt ist, wird der schlauchförmige Behälter 2b dann durch mehrere Schweißnähte 19 in unterschiedliche Kammern 20 unterteilt bzw. verschlossen.

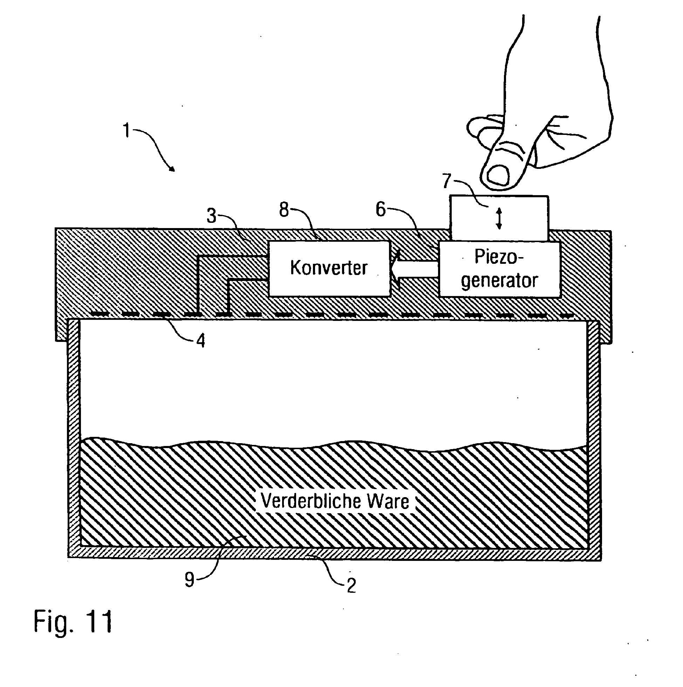

Figur 11 zeigt eine Abwandlung der Desinfektionseinrichtung gemäß Figur 1, so dass zur Vermeidung von Wiederholungen auf die vorstehende Beschreibung verwiesen wird, wobei für entsprechende Einzelheiten dieselben Bezugszeichen verwendet werden.

Eine Besonderheit dieses Ausführungsbeispiels besteht darin, dass der deckelförmige Plasmaapplikator 3 den Behälter 2 gasdicht abschließen kann, was einen Vakuumverschluss ermöglicht, wie er insbesondere in der Lebensmittelverpackungstechnik üblich ist.

Darüber hinaus ist die Plasmaquelle 4 hierbei als flächige Elektrode ausgebildet, wie beispielsweise als DBD-Elektrode oder als SMD-Elektrode.

Vorteilhaft an dieser Abwandlung ist die Tatsache, dass der in dem Behälter 2 angeordnete Gegenstand, beispielsweise das Lebensmittel 9, unter Vakuumbedingungen mit dem Plasma behandelt werden kann.

Es ist generell möglich, dass die Plasmaquelle 4 des Behälters 2 als flächige Elektrode ausgebildet ist beziehungsweise eine flächige Elektrode umfasst. Diese kann als DBD-Elektrode oder als SMD-Elektrode ausgebildet sein. Es ist auch möglich, dass die Plasmaquelle 4 nach dem Prinzip der selbststerilisierenden Oberfläche (self sterilizing surface, SSS) arbeitet. Insbesondere ist eine entsprechende Ausbildung der Plasmaquelle 4 nicht nur bei dem in Figur 11 dargestellten Ausführungsbeispiel, sondern auch bei allen anderen bevorzugten Ausführungsbeispielen der Desinfektionseinrichtung beziehungsweise des Behälters 2 möglich.

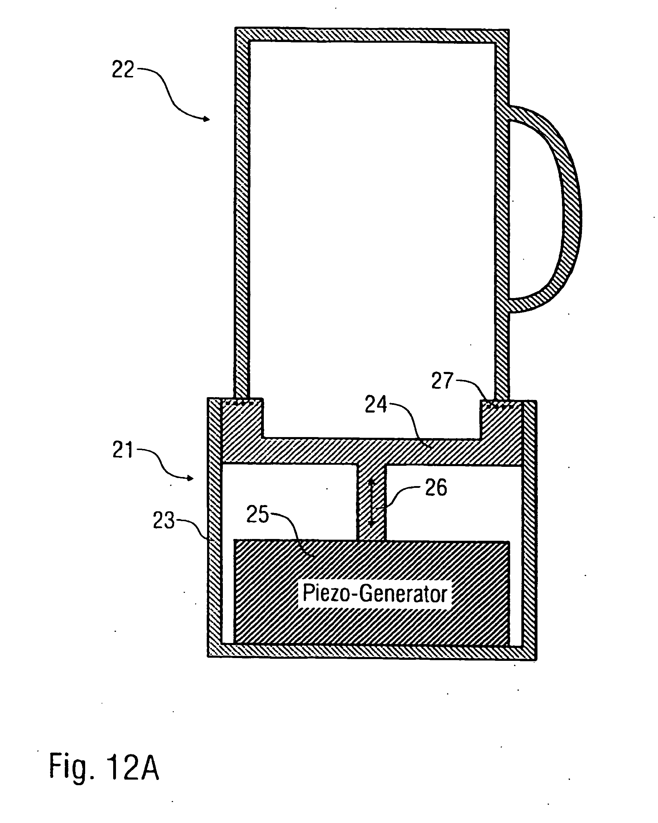

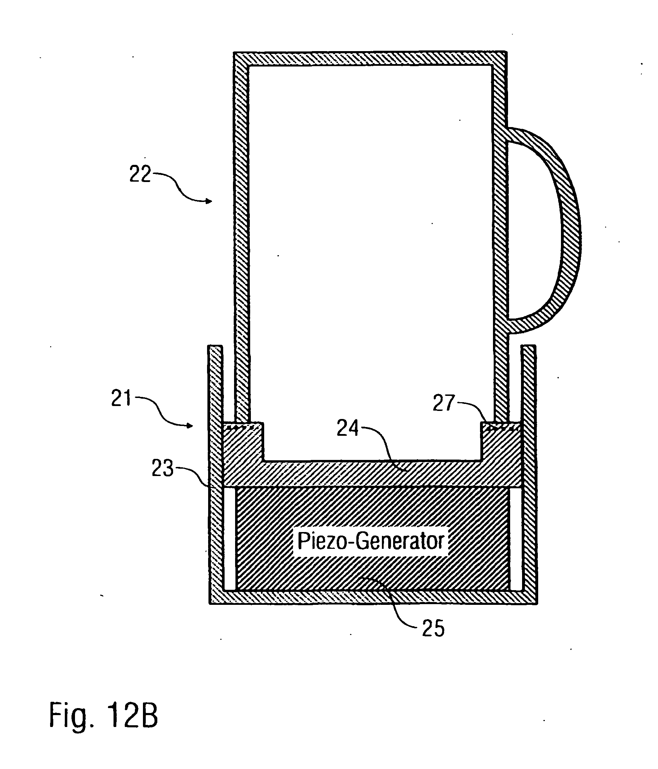

Die Figuren 12A und 12B zeigen ein Ausführungsbeispiel einer erfindungsgemäßen Desinfektionseinrichtung 21 zur Desinfektion von Getränkebehältern, insbesondere Gläsern, vor allem Trinkgläsern, Babyflaschen, oder Bierkrügen 22. Die Desinfektionseinrichtung 21 weist hierzu eine vorzugsweise wannenförmige Aufnahme 23 auf, in der ein Stempel 24 in Pfeilrichtung vertikal verschiebbar ist.

Die Desinfektionseinrichtung 21 wird hier anhand einer Verwendung mit einem Bierkrug 22 erläutert. Dies ist jedoch nicht einschränkend zu verstehen; die Desinfektionseinrichtung 21 kann vielmehr mit beliebigen Getränkebehältern, insbesondere auch mit Gläsern oder Babyflaschen verwendet werden.

Der Stempel 24 umfasst zumindest in einem Bereich, welcher mit einem Mündungsrand einen zu desinfizierenden Getränkebehälters zusammenwirkt, eine Plasmaquelle. Diese ist durch Verlagerung des Stempels 24, hier insbesondere durch dessen vertikale Verschiebung, aktivierbar. Vorzugsweise ist eine elektrische Energiequelle vorgesehen, die einerseits mit dem Stempel 24 und andererseits mit der Plasmaquelle zusammenwirkt, so dass ein Plasma im Bereich des Mündungsrands des zu desinfizierenden Behälters erzeugt wird, wenn der Stempel 24 verlagert wird.

Bei dem dargestellten Ausführungsbeispiel befindet sich in der Aufnahme 23 eine elektrische Energiequelle, hier ein Piezogenerator 25, die vorzugsweise über eine Stange 26 von dem Stempel 24 betätigt wird, wenn der Bierkrug 22 auf den Stempel 24 aufgesetzt und mit dem Stempel 24 herunter gedrückt wird. Bei dieser Bewegung erzeugt die elektrische Energiequelle, hier der Piezogenerator 25 elektrische Energie, die zum Betrieb einer Plasmaquelle 27 erforderlich ist, wobei die Plasmaquelle 27 eine flächige Elektrode (z.B. DBD-/SMD-/SSS-Elektrode) aufweist, die dann am Mündungsrand des Bierkrugs 22 ein Plasma erzeugt, um diesen zu desinfizieren.

Figur 12B zeigt ein weiteres Ausführungsbeispiel der Desinfektionseinrichtung 21. Gleiche und funktionsgleiche Elemente sind mit gleichen Bezugszeichen versehen, so dass insofern auf die vorangegangene Beschreibung verwiesen wird. In Figur 12B ist der Bierkrug 22 in seiner vertikal nach unten verlagerten Position dargstellt, in welcher auch der Stempel 24 vertikal nach unten verlagert ist. Entsprechend ist die Plasmaquelle 27 durch die elektrische Energiequelle, hier den Piezogenerator 25 aktiviert, und der Mündungsrand des Bierkrugs 22 wird desinfiziert.

Es wird deutlich, dass die Desinfektionseinrichtung und das Desinfektionsverfahren, der Behälter sowie die Verwendung des Behälters insbesondere der Verlängerung der Lebensdauer einer verderblichen Ware dienen können. Dadurch, dass ein Innenraum des Behälters vor Einlagerung der Ware desinfiziert werden kann, wird diese nicht durch eine Behälterwandung kontaminiert, wenn sie in den Behälter eingelegt wird. Zusätzlich oder alternativ ist es möglich, den in dem Behälter angeordneten Gegenstand, vorzugsweise die verderbliche Ware beziehungsweise das Lebensmittel, in dem Behälter zu desinfizieren, so dass die Keimlast auf dem Gegenstand reduziert beziehungsweise klein gehalten wird.

Es ist möglich, dass der Behälter dabei zumindest bereichsweise offen oder vollständig geschlossen ist. Besonders bevorzugt ist der Behälter durch den als Verschlussdeckel oder Verschlussstopfen ausgebildeten Plasmaapplikator dicht verschlossen.

Es ist möglich, den Innenraum des Behälters zu evakuieren, um die Plasmabehandlung durchzuführen. Insbesondere wenn der Behälter allerdings durch den als Verschlussdeckel oder Verschlussstopfen ausgebildeten Plasmaapplikator dicht verschlossen ist, und ganz besonders wenn eine der hier genannten Plasmaquellen in Zusammenhang mit dem Plasmaapplikator genutzt wird, ist es nicht nötig, den Innenraum des Behälters zu evakuieren. Die Plasmabehandlung erfolgt insbesondere in diesem Fall mit großer Wirksamkeit auch bei Normaldruck beziehungsweise Umgebungsdruck.

Es ist möglich, einen Sicherheitsmechanismus vorzusehen, der eine Aktivierung der Plasmaquelle nur bei geschlossenem Behälterdeckel und/oder anderweitig gesicherter Plasmaquelle erlaubt.

### Bezugszeichenliste:

- 1: Desinfektionseinrichtung
- 2: Behälter
- 2a: Behälter
- 2b: Behälter
- 3: Plasmaapplikator
- 4: Plasmaquelle
- 5: Austrittsöffnung
- 6: Piezogenerator
- 6a: Batterie
- 6b: Generator
- 6c: Transformator
- 7: Betätigungsglied
- 8: Konverter
- 9: Lebensmittel
- 10: Kurbel
- 11: Netzkabel
- 12: Netzstecker
- 13: Steckdose
- 14: Ringförmige Elektrodenanordnung
- 15: Dichtringe
- 16: Eintrittsöffnung
- 17: Austrittsöffnung
- 18: Einfüllöffnung
- 19: Schweißnaht
- 20: Kammer
- 21: Desinfektionseinrichtung
- 22: Bierkrug
- 23: Aufnahme
- 24: Stempel
- 25: Piezogenerator
- 26: Stange
- 27: Plasmaquelle
- V1: Ventil
- V2: Ventil
- V3: Ventil

## Patentansprüche

1. Desinfektionseinrichtung (1) zur Desinfektion eines Innenraums eines Behälters (2, 2a, 2b, 2c), der zur Aufnahme eines Gegenstands, insbesondere einer verderblichen Ware (9) dient, bevorzugt eines Lebensmittelbehälters, mit einem Plasmaapplikator (3) zur Applikation eines nicht-thermischen, atmosphärischen Plasma in den Innenraum des Behälters (2, 2a, 2b, 2c), **dadurch gekennzeichnet, dass** der Plasmaapplikator (3) in seiner äußeren Form einem Verschlussdeckel oder einem Verschlussstopfen für einen Lebensmittelbehälter (2), insbesondere für eine Flasche, entspricht, wobei der Plasmaapplikator (3) so ausgebildet ist, dass er dauerhaft auf dem Lebensmittelbehälter (2) belassen werden kann, wobei der Lebensmittelbehälter (2) durch den Plasmaapplikator (3) dicht verschlossen ist.

2. Desinfektionseinrichtung (1) nach Anspruch 1,
**dadurch gekennzeichnet,**
a) **dass** der Plasmaapplikator (3) einen lösbaren Verschluss für eine Behälteröffnung des Behälters (2, 2a, 2b, 2c) bildet, und/oder
b) **dass** der Plasmaapplikator (3) an der Innenseite des Verschlusses eine Austrittsöffnung (5) aufweist, um das nicht-thermische, atmosphärische Plasma in den Innenraum des Behälters (2, 2a, 2b, 2c) abzugeben, während der Plasma-applikator (3) die Behälteröffnung verschließt.

3. Desinfektionseinrichtung (1) nach Anspruch 2,
**dadurch gekennzeichnet,**
a) **dass** der Plasmaapplikator (3) einen Verschlussdeckel bildet, der auf die Behälteröffnung aufsetzbar ist, und/oder
b) **dass** der als Verschlussdeckel ausgebildete Plasmaapplikator (3) einen Vakuumverschluss des Behälters ermöglicht, und/oder
c) **dass** der als Verschlussdeckel ausgebildete Plasmaapplikator (3) zur Erzeugung des Plasmas eine integrierte Plasma-elektrode aufweist, und/oder
d) **dass** das Plasma in dem Behälter ohne einen Gasaustausch mit der Umgebung erzeugt werden kann.

4. Desinfektionseinrichtung (1) nach Anspruch 3,
**dadurch gekennzeichnet,**
a) **dass** der als Verschlussdeckel ausgebildete Plasmaapplikator (3) einen Außenquerschnitt aufweist, der sich in Bezug auf den Behälter (2, 2a, 2b, 2c) von außen nach innen verjüngt, so dass der Verschlussdeckel auf verschieden große Behälteröffnungen passt, und
b) vorzugsweise, dass der als Verschlussdeckel ausgebildete Plasmaapplikator (3) einen trapezförmigen Querschnitt aufweist.

5. Desinfektionseinrichtung (1) nach Anspruch 2, **dadurch gekennzeichnet, dass** der Plasmaapplikator (3) als Verschlusstopfen ausgebildet ist, der in die Behälteröffnung einführbar ist.

6. Desinfektionseinrichtung (1) nach einem der vorhergehenden Ansprüche, **dadurch gekennzeichnet,**
a) **dass** der Plasmaapplikator (3) eine ringförmige Elektrodenanordnung (14) zur Erzeugung des Plasmas aufweist, und
b) **dass** die ringförmige Elektrodenanordnung (14) entlang dem Umfangsrand der Behälteröffnung verläuft, und
c) **dass** die ringförmige Elektrodenanordnung (14) das Plasma erzeugt, so dass das Plasma den Umfangsrand der Behälteröffnung desinfiziert.

7. Desinfektionseinrichtung (1) nach einem der vorhergehenden Ansprüche, **dadurch gekennzeichnet, dass** der Plasmaapplikator (3) folgende Komponenten aufweist, die in den Plasmaapplikator (3) integriert sind:
a) eine elektrisch betriebene Plasmaquelle (4) zur Erzeugung des nicht-thermischen atmosphärischen Plasmas, und
b) eine elektrische Energiequelle (6, 6a, 6b, 6c) zur Bereitstellung der zum Betrieb der Plasmaquelle (4) erforderlichen elektrischen Energie.

8. Desinfektionseinrichtung (1) nach Anspruch 7, **dadurch gekennzeichnet, dass** die elektrische Energiequelle (6, 6a, 6b, 6c) folgende Komponenten aufweist, die in den Plasmaapplikator (3) integriert sind:
a) einen Piezogenerator (6) zur Erzeugung der zum Betrieb der Plasmaquelle (4) erforderlichen elektrischen Energie durch eine mechanische Betätigung des Piezogenerators (6), und
b) vorzugsweise einen Konverter (8), der eingangsseitig mit dem Piezogenerator (6) und ausgangsseitig mit der Plasmaquelle (4) verbunden ist, wobei der Konverter (8) die von dem Piezogenerator (6) gelieferte elektrische Energie in die für den Betrieb der Plasmaquelle (4) benötige elektrische Energie umwandelt, und
c) vorzugsweise ein manuelles Betätigungsglied (7), das mechanisch mit dem Piezogenerator (6) gekoppelt ist, so dass der Piezogenerator (6) manuell betätigt werden kann, um die zum Betrieb der Plasmaquelle (4) erforderliche elektrische Energie zu erzeugen.

9. Desinfektionseinrichtung (1) nach Anspruch 7 oder 8, **dadurch gekennzeichnet, dass** die elektrische Energiequelle mindestens eine der folgenden Komponenten aufweist:
a) einen Generator (6b),
b) eine Batterie (6a), insbesondere eine wiederaufladbare Batterie,
c) einen Netzanschluss (11, 12), der mit einem elektrischen Stromnetz (13)verbunden werden kann,
d) eine Solarzelle,
e) eine Energy-Harvesting-Energiequelle, insbesondere einen Piezogenerator (6).

10. Behälter, insbesondere zur Aufbewahrung verderblicher Ware, vorzugsweise von Lebensmitteln, mit einer Behälteröffnung, **dadurch gekennzeichnet, dass** der Behälter einen die Behälteröffnung verschließenden Verschlussdeckel oder Verschlussstopfen umfasst, der als Desinfektionseinrichtung nach einem der Ansprüche 1 bis 9 ausgebildet ist.

11. Verwendung eines Behälters nach Anspruch 10 zur Reduzierung einer Keimlast auf einem in dem Behälter angeordneten Gegenstand, insbesondere auf verderblicher Ware, bevorzugt auf Lebensmitteln.

12. Verwendung eines Behälters nach Anspruch 10 zur Lagerung und/oder zum Transport eines Gegenstands, insbesondere von verderblicher Ware, bevorzugt von Lebensmitteln.

## Claims

1. Disinfection device (1) for disinfection of an internal space of a container (2, 2a, 2b, 2c) serving for accommodation of an object, in particular a perishable goods (9), preferably a food container, having a plasma applicator (3) for application of a non-thermal atmospheric plasma into the internal space of the container (2, 2a, 2b, 2c), **characterised in that** the external shape of the plasma applicator (3) is equivalent to a closure lid or a closure stopper for a food container (2), in particular for a bottle, whereby the plasma applicator (3) is formed appropriately such that it can be left permanently on the food container (2), whereby the food container (2) is closed tightly by the plasma applicator (3).

2. Disinfection device (1) according to claim 1,
**characterised in that**
a) the plasma applicator (3) forms a detachable closure for a container opening of the container (2, 2a, 2b, 2c), and/or
b) the plasma applicator (3) comprises an exit opening (5) on the internal surface of the closure for dispensing the non-thermal atmospheric plasma into the internal space of the container (2, 2a, 2b, 2c) while the plasma applicator (3) closes the container opening.

3. Disinfection device (1) according to claim 2,
**characterised in that**
a) the plasma applicator (3) forms a closure lid that can be placed on the container opening, and/or
b) the plasma applicator (3) formed as a closure lid enables vacuum-tight closure of the container, and/or
c) the plasma applicator (3) formed as a closure lid comprises an integrated plasma electrode for generating the plasma, and/or
d) the plasma can be generated in the container in the absence of a gas exchange with the surroundings.

4. Disinfection device (1) according to claim 3,
**characterised in that**
a) the plasma applicator (3) formed as a closure lid comprises an external cross-section, which tapers from outside to inside with respect to the container (2, 2a, 2b, 2c) such that the closure lid fits on container openings of different sizes, and
b) the plasma applicator (3) formed as a closure lid preferably has a trapezoidal cross-section.

5. Disinfection device (1) according to claim 2, **characterised in that** the plasma applicator (3) is formed as a closure stopper that can be inserted into the container opening.

6. Disinfection device (1) according to any one of the preceding claims, **characterised in that**
a) the plasma applicator (3) comprises a ring-shaped electrode arrangement (14) for generating the plasma, and
b) the ring-shaped electrode arrangement (14) extends along the circumferential edge of the container opening, and
c) the ring-shaped electrode arrangement (14) generates the plasma such that the plasma disinfects the circumferential edge of the container opening.

7. Disinfection device (1) according to any one of the preceding claims, **characterised in that** the plasma applicator (3) comprises the following components that are integrated into the plasma applicator (3):
a) an electrically operated plasma source (4) for generating the non-thermal atmospheric plasma, and
b) a source of electrical energy (6, 6a, 6b, 6c) for providing the electrical energy required for operation of the plasma source (4).

8. Disinfection device (1) according to claim 7, **characterised in that** the source of electrical energy (6, 6a, 6b, 6c) comprises the following components that are integrated into the plasma applicator (3):
a) a piezo generator (6) for generating the electrical energy required for operation of the plasma source (4) through a mechanical actuation of the piezo generator (6), and
b) preferably, a converter (8) that is connected to the piezo generator (6) on the input side and is connected to the plasma source (4) on the output side, whereby the converter (8) converts the electrical energy supplied by the piezo generator (6) into the electrical energy required for operation of the plasma source (4), and
c) preferably, a manual actuation element (7) that is mechanically coupled to the piezo generator (6) such that the piezo generator (6) can be actuated manually in order to generate the electrical energy required to operate the plasma source (4).

9. Disinfection device (1) according to claim 7 or 8, **characterised in that** the source of electrical energy comprises at least one of the following components:
a) a generator (6b);
b) a battery (6a), in particular a rechargeable battery;
c) a connection (11, 12) to the supply system that can be connected to an electrical power supply system (13);
d) a solar cell;
e) an energy-harvesting energy source, in particular a piezo generator (6).

10. Container, in particular for storage of perishable goods, preferably of food items, having a container opening, **characterised in that** the container comprises a closure lid or closure stopper that closes the container opening and is formed as disinfection device according to any one of the claims 1 to 9.

11. Use of a container according to claim 10 for reducing a germ load on an object arranged in said container, in particular on perishable goods, preferably on food items.

12. Use of a container according to claim 10 for storage and/or transport of an object, in particular of perishable goods, preferably of food items.

## Revendications

1. Dispositif de désinfection (1) pour la désinfection d'un espace interne d'un récipient (2, 2a, 2b, 2c) qui sert à recevoir un objet, notamment une denrée périssable (9), de préférence d'un récipient alimentaire, **comprenant** un applicateur plasma (3) pour l'application d'un plasma atmosphérique non thermique dans l'espace interne du récipient (2, 2a, 2b, 2c), **caractérisé en ce que** l'applicateur plasma (3) correspond dans sa forme extérieure à un couvercle de fermeture ou un bouchon de fermeture pour un récipient alimentaire (2), notamment pour une bouteille, dans lequel l'applicateur plasma (3) est réalisé de sorte qu'il peut être laissé de manière durable sur le récipient alimentaire (2), dans lequel le récipient alimentaire (2) est fermé de manière étanche par l'applicateur plasma (3).

2. Dispositif de désinfection (1) selon la revendication 1, **caractérisé en ce**
a) **que** l'applicateur plasma (3) forme une fermeture amovible pour une ouverture de récipient du récipient (2, 2a, 2b, 2c), et/ou
b) **que** l'applicateur plasma (3) présente sur le côté interne de la fermeture une ouverture de sortie (5) pour distribuer le plasma atmosphérique non thermique dans l'espace interne du récipient (2, 2a, 2b, 2c) tandis que l'applicateur plasma (3) ferme l'ouverture de récipient.

3. Dispositif de désinfection (1) selon la revendication 2, **caractérisé en ce**
a) **que** l'applicateur plasma (3) forme un couvercle de fermeture qui peut être posé sur l'ouverture de récipient, et/ou
b) **que** l'applicateur plasma (3) réalisé en tant que couvercle de fermeture permet une fermeture sous vide du récipient, et/ou
c) **que** l'applicateur plasma (3) réalisé en tant que couvercle de fermeture présente une électrode plasma intégrée pour la génération du plasma, et/ou
d) **que** le plasma dans le récipient peut être généré sans échange gazeux avec l'environnement.

4. Dispositif de désinfection (1) selon la revendication 3, **caractérisé en ce**
a) **que** l'applicateur plasma (3) réalisé en tant que couvercle de fermeture présente une section transversale externe qui rétrécit de l'extérieur vers l'intérieur par rapport au récipient (2, 2a, 2b, 2c) de sorte que le couvercle de fermeture s'adapte à des ouvertures de récipient de différentes tailles, et
b) de préférence, que l'applicateur plasma (3) réalisé en tant que couvercle de fermeture présente une section transversale trapézoïdale.

5. Dispositif de désinfection (1) selon la revendication 2, **caractérisé en ce que** l'applicateur plasma (3) est réalisé en tant que bouchon de fermeture qui peut être introduit dans l'ouverture de récipient.

6. Dispositif de désinfection (1) selon une des revendications précédentes, **caractérisé en ce**
a) **que** l'applicateur plasma (3) présente une disposition d'électrode annulaire (14) pour la génération du plasma, et
b) **que** la disposition d'électrode annulaire (14) s'étend le long du bord périphérique de l'ouverture de récipient, et
c) **que** la disposition d'électrode annulaire (14) génère le plasma de sorte que le plasma désinfecte le bord périphérique de l'ouverture de récipient.

7. Dispositif de désinfection (1) selon une des revendications précédentes, **caractérisé en ce que** l'applicateur plasma (3) présente les composants suivants qui sont intégrés dans l'applicateur plasma (3) :
a) une source de plasma à fonctionnement électrique (4) pour la génération du plasma atmosphérique non thermique, et
b) une source d'énergie électrique (6, 6a, 6b, 6c) pour la mise à disposition de l'énergie électrique nécessaire au fonctionnement de la source de plasma (4).

8. Dispositif de désinfection (1) selon la revendication 7, **caractérisé en ce que** la source d'énergie électrique (6, 6a, 6b, 6c) présente les composants suivants qui sont intégrés dans l'applicateur plasma (3) :
a) un générateur piézoélectrique (6) pour la génération de l'énergie électrique nécessaire au fonctionnement de la source de plasma (4) par un actionnement mécanique du générateur piézoélectrique (6), et
b) de préférence un convertisseur (8) qui est relié côté entrée au générateur piézoélectrique (6) et côté sortie à la source de plasma (4), dans lequel le convertisseur (8) transforme l'énergie électrique livrée par le générateur piézoélectrique (6) en l'énergie électrique nécessaire au fonctionnement de la source de plasma (4), et
c) de préférence un organe d'actionnement manuel (7) qui est raccordé mécaniquement au générateur piézoélectrique (6) de sorte que le générateur piézoélectrique (6) peut être actionné manuellement pour générer l'énergie électrique nécessaire au fonctionnement de la source de plasma (4).

9. Dispositif de désinfection (1) selon la revendication 7 ou 8, **caractérisé en ce que** la source d'énergie électrique présente au moins un des composants suivants :
a) un générateur (6b),
b) une batterie (6a), notamment une batterie rechargeable,
c) un raccordement au réseau (11, 12) qui peut être relié à un réseau de courant électrique (13),
d) une cellule solaire,
e) une source d'énergie à récupération d'énergie, notamment un générateur piézoélectrique (6).

10. Récipient, notamment pour la conservation d'une denrée périssable, de préférence d'aliments, avec une ouverture de récipient, **caractérisé en ce que** le récipient comprend un couvercle de fermeture ou bouchon de fermeture fermant l'ouverture de récipient qui est réalisé en tant que dispositif de désinfection selon une des revendications 1 à 9.

11. Utilisation d'un récipient selon la revendication 10 pour la réduction d'une charge bactérienne sur un objet disposé dans le récipient, notamment sur une denrée périssable, de préférence sur des aliments.

12. Utilisation d'un récipient selon la revendication 10 pour l'entreposage et/ou pour le transport d'un objet, notamment d'une denrée périssable, de préférence d'aliments.
